# EUROPEAN PATENT APPLICATION

(11) **EP 4 628 212 A1**
(43) Date of publication of application: **08.10.2025**
(21) Application number: 23898409.0
(22) Date of filing: 04.12.2023
(51) Int. Cl.: B01L 3/00

(54) **ANALYTE MONITORING DEVICE**

(30) Priority: 02.12.2022 KR 20220166654
(71) Applicant: SD Biosensor, Inc., Gyeonggi-do 16690 (KR)
(72) Inventor: PARK, Hyo-Lim, Suwon-si, Gyeonggi-do 16684 (KR); LEE, Sang Min, Suwon-si, Gyeonggi-do 16690 (KR); LEE, Hyo Keun, Suwon-si, Gyeonggi-do 16690 (KR)
(74) Representative: J A Kemp LLP
(86) International application number: PCT/KR2023/019815
(87) International publication number: WO 2024/117886

(57) **Abstract**

Provided is an analyte monitoring device including an upper housing, a sensor unit inserted into a body, a printed circuit board (PCB) unit which is spaced apart from an upper housing and on which the sensor unit is mounted, an antenna unit formed on the upper housing, and a conduction unit disposed between the PCB unit and the antenna unit and allowing the PCB unit and the antenna unit to be electrically connected to each other.

## Description

### [Technical Field]

The present disclosure relates to an analyte monitoring device, and more particularly, to a device that is inserted into the body and used for monitoring an analyte.

### [Background Art]

Contents described in this section merely provide background information for the present disclosure and do not constitute prior art.

As the taste for processed foods has increased, people are easily exposed to monosaccharides. Due to this environment, diabetes is also rapidly increasing. When blood glucose levels drop rapidly or rise rapidly, diabetic patients can experience shock, and in rare cases, death. Thus, diabetic patients need to continuously monitor their blood glucose levels.

In the case of blood glucose measurement devices according to the related art, a lancet is used to cut the tip of the finger, and the blood of the diabetic patient that comes out through a cut gap is inserted into a blood glucose analysis device to calculate blood glucose level.

This type of blood glucose measurement device causes pain and fear to a user. In addition, it is realistically difficult to cut one's finger every hour to cause bleeding.

In order to solve this problem, a continuous glucose monitoring (CGM) device has been developed. When a sensor having microscopic thickness and an electronic apparatus for analyzing an analyte measured in the sensor (hereinafter, "blood glucose") are attached to the skin of the diabetic patient, blood glucose levels are continuously measured for a period of one week to ten days.

The continuous glucose monitoring (CGM) device can measure blood glucose levels continuously using the sensor that is inserted into the skin of the body and provided in the device. In general, efforts have been made to miniaturize a CGM device so that the CGM device can be inserted into the skin of the body. However, in CGM devices according to the related art, it is difficult to control the strength and signal characteristics of an antenna during a product miniaturization process.

### (Prior art Document)

### (Patent Document)

Korean Patent Registration No. 10-2566020 (August 10, 2023)
Korean Patent Registration No. 10-2593526 (October 19, 2023)

### [Disclosure]

### [Technical Problem]

The present disclosure provides an analyte monitoring device that secures the strength of an antenna and signal characteristics through an antenna unit formed separately from a printed circuit board (PCB) unit and finds out the user's blood glucose information.

The problems to be solved by the present disclosure are not limited to the problems mentioned above, and other problems not mentioned will be clearly understood by those skilled in the art from the description below.

### [Technical Solution]

According to an aspect of the present disclosure, there is provided an analyte monitoring device including: an upper housing; a sensor unit inserted into a body; a printed circuit board (PCB) unit which is spaced apart from an upper housing and on which the sensor unit is mounted; an antenna unit formed on the upper housing; and a conduction unit disposed between the PCB unit and the antenna unit and allowing the PCB unit and the antenna unit to be electrically connected to each other.

A first opening may be formed in the upper housing, and a fixing unit coupled to the sensor unit may be fixed in the first opening, and the fixing unit may include a holder covering part of the first opening and coupled to the sensor unit, and a packing holder covering the other part of the first opening and coupled to the holder.

The packing holder may include a packing holder sensor fixing portion formed with a width corresponding to a width of the sensor unit, and the holder may include a holder sensor fixing portion formed with a width corresponding to the width of the sensor unit.

The packing holder may further include a packing holder protrusion formed at one end of the packing holder in a protrusion structure, and the holder may further include a holder insertion portion in a structure corresponding to the packing holder protrusion.

The packing holder may be manufactured of a rubber material.

Predetermined patterns may be formed on the antenna unit using a laser direct structuring (LDS) method.

The sensor unit may include: a sensor body portion coupled to the PCB unit; and a sensor insertion portion vertically extending from the sensor body portion and inserted into the body.

The conduction unit may include: an antenna contact portion in contact with the antenna unit; a PCB contact portion disposed parallel to the antenna contact portion and in contact with the PCB unit; and a conduction connection portion connecting the antenna contact portion to the PCB contact portion.

The analyte monitoring device may further include a lower housing coupled to the upper housing, aligned with the first opening and including at least one second opening through which part of the sensor unit passes.

### [Advantageous Effects]

In an analyte monitoring device according to the present disclosure, a sensor unit is inserted into the body and finds out the user's blood glucose information continuously so that the user's body information can be found out in real time.

In the analyte monitoring device according to the present disclosure, the strength of an antenna and signal characteristics can be secured by an antenna unit formed separately from a printed circuit board (PCB) unit, and blood glucose information measured by the sensor unit can be transmitted/received to/from an external device.

In the analyte monitoring device according to the present disclosure, even when a sensor is inserted into the body, a fixing unit can fix the sensor so that the sensor cannot move.

In the analyte monitoring device according to the present disclosure, when a packing holder is formed of a rubber material and the analyte monitoring device is attached to the body using an applicator, a friction force is increased to prevent the analyte monitoring device from being rotated, and a hole formed by an applicator needle can be automatically restored through the rubber material.

### [Brief Description of the Drawing]

FIG. 1 is a perspective view of an analyte monitoring device according to an embodiment of the present disclosure;
FIG. 2 is a bottom perspective view of the analyte monitoring device according to an embodiment of the present disclosure;
FIG. 3 is an exploded perspective view of the analyte monitoring device according to an embodiment of the present disclosure;
FIG. 4 is a perspective view of patterns of an antenna unit according to an embodiment of the present disclosure;
FIG. 5 is a perspective view illustrating the inside of the analyte monitoring device according to an embodiment of the present disclosure;
FIG. 6 is an internal cross-sectional view of the analyte monitoring device according to an embodiment of the present disclosure;
FIG. 7 is an enlarged view illustrating a state in which a conductive unit according to an embodiment of the present disclosure is in contact with the antenna unit and a printed circuit board (PCB) unit; and
FIG. 8 is a bottom perspective view illustrating a fixing unit according to an embodiment of the present disclosure.

### [Detailed Description of Preferred Embodiments]

Hereinafter, some embodiments of the present disclosure will be described in detail through exemplary drawings. When adding reference numerals to components in each drawing, it should be noted that the same components have the same reference numerals as much as possible even if they are shown in different drawings. Also, in describing the present disclosure, if it is determined that a detailed description of a related known configuration or function may obscure the gist of the present disclosure, the detailed description will be omitted.

In describing the components of the embodiment according to the present disclosure, symbols, such as first, second, i), ii), a), and b), and the like may be used. These symbols are only used to distinguish the component from other components, and the nature, sequence, or order of the component is not limited by the symbols. In the specification, when a part is said to 'include' or 'comprise' a certain element, this means that it does not exclude other elements, but may further include other elements, unless explicitly stated to the contrary.

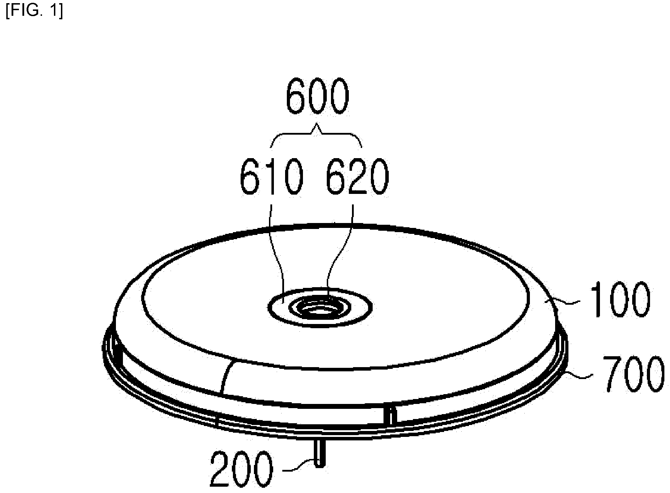

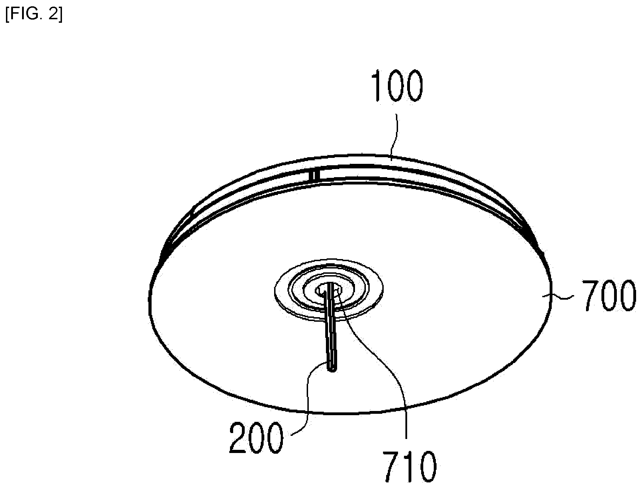

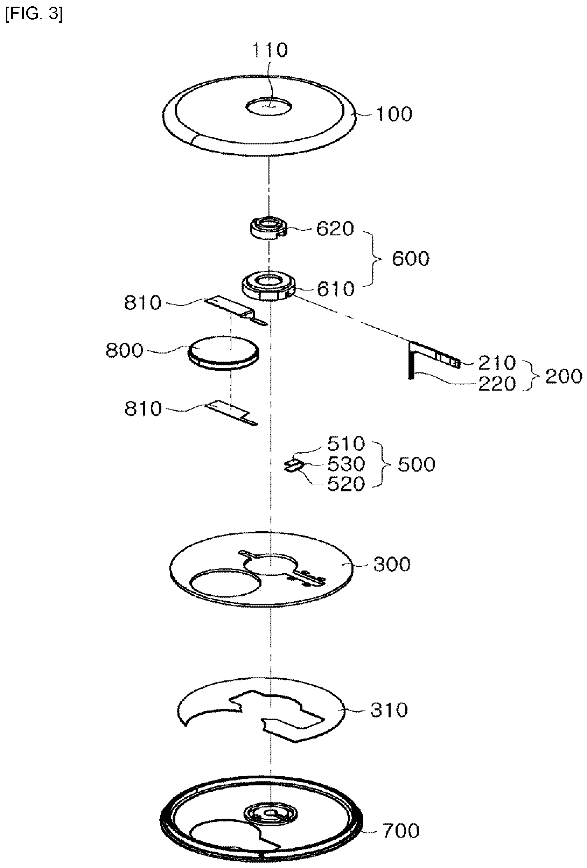

FIG. 1 is a perspective view of an analyte monitoring device according to an embodiment of the present disclosure, FIG. 2 is a bottom perspective view of the analyte monitoring device according to an embodiment of the present disclosure, and FIG. 3 is an exploded perspective view of the analyte monitoring device according to an embodiment of the present disclosure.

Referring to FIGS. 1 to 3, the analyte monitoring device according to the present embodiment includes an upper housing 100, a sensor unit 200, a printed circuit board (PCB) unit 300, an antenna unit 400, and a conduction unit 500.

The upper housing 100 may protect components inside the analyte monitoring device. The upper housing 100 is pressed by an applicator, i.e., a tool on which the analyte monitoring device is mounted to insert the sensor unit 200 of the analyte monitoring device into a user's body, and the analyte monitoring device is attached to the body by pressurization. The upper housing 100 is preferably formed of a synthetic resin, for example, plastic, and the present disclosure is not limited thereto, and the upper housing 100 may be manufactured of various materials.

The sensor unit 200 measures the analyte in the body. The analyte measured by the sensor unit 200 may include a biological material including glucose, etc. The sensor unit 200 may be mounted on the PCB unit 300 to be described later and may measure the analyte, and the measured information may be transmitted/received to/from an external device through the PCB unit 300 to be described later and the antenna unit 400 to be described later.

Referring to FIG. 3, the sensor unit 200 may include a sensor body portion 210 and a sensor insertion portion 220.

The sensor body portion 210 is a portion of the sensor unit 200 coupled to the PCB unit 300. The sensor body portion 210 may transmit a sensor detection signal measured by the sensor insertion portion 220 to be described later to the PCB unit 300. The sensor body portion 210 is shown in a hexahedron shape in FIG. 3, but the present disclosure is not limited thereto, and the sensor body portion 210 may be manufactured in various shapes.

The sensor insertion portion 220 is a portion of the sensor unit 200 that is inserted into the body and measures information about the analyte inside the body. The sensor insertion portion 220 may be inserted into the body and may measure information about the analyte inside the body, and the measured information may be transmitted to the PCB unit 300 through the sensor body portion 210. The sensor insertion portion 220 may extend from the sensor body portion 210 vertically downward and may be inserted in a direction perpendicular to the surface of the skin. When the sensor insertion portion 220 does not extend perpendicular to the sensor body portion 210, as the sensor unit 200 (specifically, a connection portion of the sensor body portion and the sensor insertion portion) is bent while the sensor insertion portion 220 is pressed by the applicator, there is a risk that a fine electrode formed on the sensor unit 200 may be broken. Since the sensor insertion portion 220 of the present disclosure extends vertically from the sensor body portion 210, the connection portion of the sensor insertion portion 220 and the sensor body portion 210 is not bent even during the pressing process of the applicator, and thus, the risk of breakage of the sensor provided in the sensor insertion portion 220 can be prevented.

The PCB unit 300 is spaced apart from the upper housing 100 and performs electrical connection between components mounted on the PCB unit 300 and the sensor unit 200. The sensor unit 200 may be mounted on the PCB unit 300. The information about the analyte (which may have the shape of electrical signals) measured by the sensor unit 200 may be transmitted to the PCB unit 300, and the PCB unit 300 may process electrical signals transmitted using a certain method. In FIG. 3, the PCB unit 300 is shown in a circular shape, but the present disclosure is not limited thereto, and the PCB unit 300 may be formed in various shapes.

A PCB pad 310 may be provided between a lower end of the PCB unit 300 and the lower housing 700 to be described later. The PCB pad 310 can prevent the PCB unit 300 from being broken by shock. The PCB pad 310 may be manufactured of various materials that may alleviate shock, such as rubber, etc..

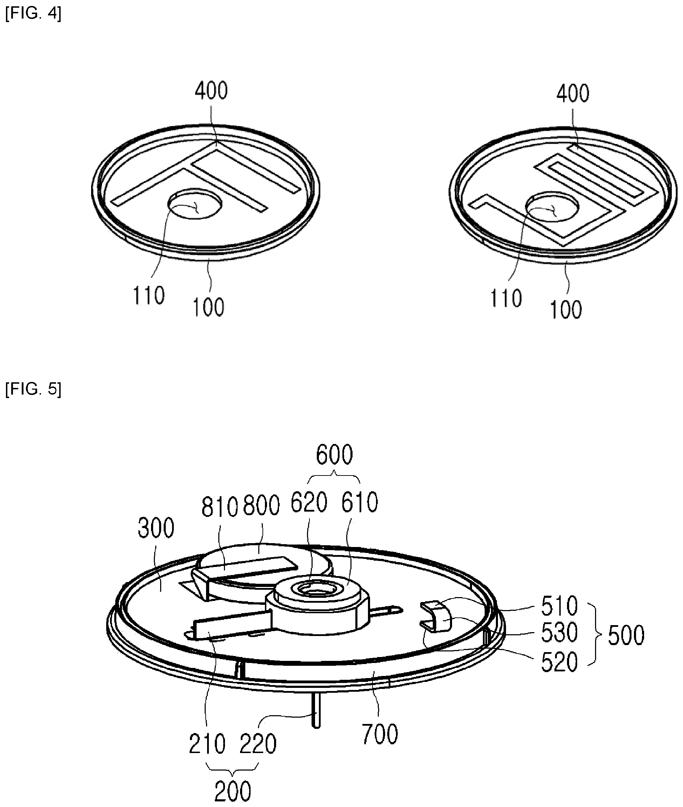

FIG. 4 is a perspective view of patterns of an antenna unit according to an embodiment of the present disclosure.

Referring to FIGS. 3 and 4, the antenna unit 400 is formed at an inner surface of the upper housing 100 and transmits/receives processed electrical signals (i.e., blood glucose information) to/from the external device. The electrical signals that correspond to the information about the analyte measured by the sensor unit 200 are transmitted to the PCB unit 300, and the conduction unit 500 to be described later allows the PCB unit 300 and the antenna unit 400 to be electrically connected to each other. The analyte information may be transmitted/received to/from the external device through the antenna unit 400 electrically connected to the PCB unit 300. As a method of transmitting/receiving the analyte information to the external device, the information is transmitted/received using mmWave or Industrial Scientific Medical (ISM) band broadband electromagnetic waves, but the present disclosure is not limited thereto, and various transmission/receiving methods may be used.

Predetermined patterns may be formed on the antenna unit 400 using a laser direct structuring (LDS) method. The LDS method is a method by which patterns are selectively processed on a thermoplastic resin using laser and then electrical characteristics and reliability are secured through a plating process. Predetermined patterns may be formed on the antenna unit 400 at part of a surface of the PCB unit 300 of the upper housing 100 using the LDS method. In general, when the antenna is mounted integrally on the PCB while a continuous glucose measurement device is miniaturized, it is difficult to adjust the strength of the antenna and signal characteristics. According to the present disclosure, the antenna unit 400 is separately formed on the upper housing 100, and predetermined patterns are processed on the upper housing 100 using the LDS method so that an appropriate strength of the antenna and signal characteristics can be secured. The patterns of the antenna unit 400 are formed in a 'F'-shape and a ' 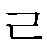 '-shape in FIG. 4, but the present disclosure is not limited thereto, and the antenna unit 400 may be formed in various patterns.

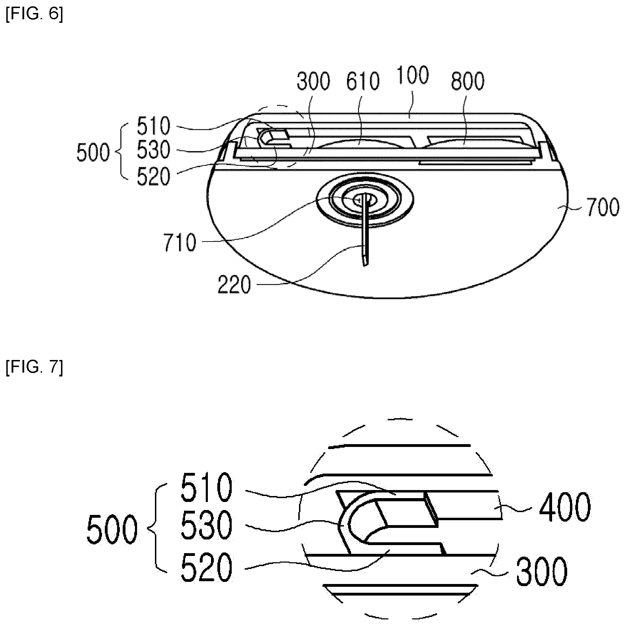

FIG. 5 is a perspective view illustrating the inside of the analyte monitoring device according to an embodiment of the present disclosure, FIG. 6 is an internal cross-sectional view of the analyte monitoring device according to an embodiment of the present disclosure, and FIG. 7 is an enlarged view illustrating a state in which a conductive unit according to an embodiment of the present disclosure is in contact with the antenna unit and a printed circuit board (PCB) unit.

Referring to FIGS. 5 to 7, the conduction unit 500 is disposed between the PCB unit 300 and the antenna unit 400 so that the PCB unit 300 and the antenna unit 400 are electrically connected to each other. The conduction unit 500 may be in contact with the antenna unit 400 formed on the upper housing 100 and simultaneously may be in contact with the PCB unit 300. The conduction unit 500 is simultaneously in contact with the antenna unit 400 and the PCB unit 300 so that electrical signals of the PCB unit 300 can be transmitted to the antenna unit 400. The conduction unit 500 may be various components formed of a conductor, and for example, a C-clip, conductive rubber, etc.

The conduction unit 500 may include an antenna contact portion 510, a PCB contact portion 520, and a conduction connection portion 530.

The antenna contact portion 510 is a portion of the conduction unit 500 that contacts the antenna unit 400. The antenna contact portion 510 may allow the PCB unit 300 and the antenna unit 400 to be electrically connected to each other through only contact with part of the patterns of the antenna unit 400 without being in contact with the whole patterns of the antenna unit 400.

The PCB contact portion 520 is disposed parallel to the antenna contact portion 510 and is a portion of the conduction unit 500 that is in contact with the PCB unit 300. The PCB contact portion 520 is disposed parallel to the antenna contact portion 510, and a distance between the upper housing 100 and the PCB unit 300 is minimized so that the size of the analyte monitoring device can be miniaturized. As shown in FIGS. 5 through 7, the antenna contact portion 510 and the PCB contact portion 520 may have hexahedron shapes, but the present disclosure is not limited thereto, and the antenna contact portion 510 and the PCB contact portion 520 may be implemented in various shapes.

The conduction connection portion 530 is a portion that connects the antenna contact portion 510 to the PCB contact portion 520. Referring to FIGS. 5 through 7, the conduction connection portion 530 may include a curved shape, but the present disclosure is not limited thereto, and the conduction connection portion 530 may be implemented in various shapes.

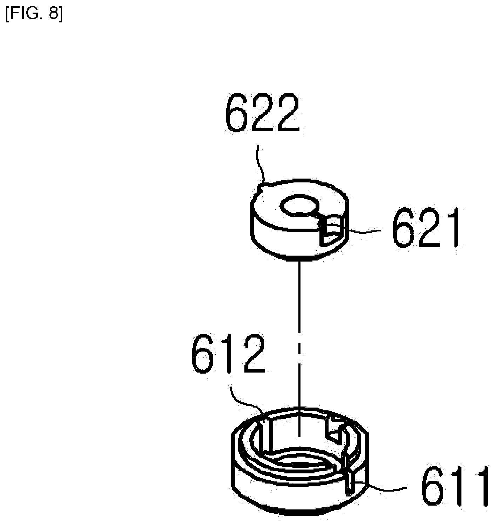

FIG. 8 is a bottom perspective view illustrating a fixing unit according to an embodiment of the present disclosure.

Referring to FIG. 8, a first opening 110 may be formed in the upper housing 100, and a fixing unit 600 coupled to the sensor unit 200 may be installed at the first opening 110.

The fixing unit 600 may be coupled to the sensor unit 200, may prevent the sensor unit 200 from being moved, and may include a holder 610 and a packing holder 620.

The holder 610 may cover part of the first opening 110 formed in the upper housing 100, may be coupled to the sensor unit 200 and may prevent the sensor unit 200 from being moved. The diameter of the holder 610 may be formed greater than the packing holder 620 to be described later, and the packing holder 620 to be described later may be coupled to the holder 610.

The holder 610 may include a holder sensor fixing portion 611 and a holder insertion portion 612.

The holder sensor fixing portion 611 may be formed by being recessed on one side of the outer surface of the holder sensor fixing portion 611 with a width corresponding to the width of the sensor unit 200, and the sensor unit 200 may be inserted into the holder sensor fixing portion 611 to fix the sensor unit 200. The holder sensor fixing portion 611 fixes the sensor unit 200 inserted into the body so that the sensor unit 200 cannot be moved at normal time and thus the user can use the analyte monitoring device safely.

The holder insertion portion 612 may be formed in a groove shape in a structure corresponding to a packing holder protrusion 622 to be described later, and the packing holder 620 to be described later may be fixed.

The packing holder 620 may cover the other part of the first opening 110 and may be coupled to the holder 610. The packing holder 620 may be pressed when a needle embedded in the applicator passes through the packing holder 620. The packing holder 620 may be manufactured of a rubber material. When the packing holder 620 is manufactured of rubber, a frictional force with the needle embedded in the applicator may be increased so that the needle can be prevented from being rotated while the analyte monitoring device is attached to the body. In addition, when the packing holder 620 is manufactured of rubber, a hole filling function may be performed by restoring a hole formed by the needle embedded in the applicator by elasticity of rubber. However, the packing holder 620 is not limited to the rubber material but may be implemented with various materials that may perform a rotation prevention function or a hole filling function.

The packing holder 620 may include a packing holder sensor fixing portion 621 and a packing holder protrusion 622. The packing holder sensor fixing portion 621 may be formed with a width corresponding to the width of the sensor unit 200 and may fix the sensor unit 200. The packing holder protrusion 622 may have a protrusion structure formed at one end of the packing holder 620 so that the packing holder 620 can be coupled to the holder 610.

According to an embodiment of the present disclosure, the packing holder 620 is coupled to the holder 610, but the present disclosure is not limited thereto, and the holder 610 and the packing holder 620 may also be integrally manufactured. In this case, the holder 610 and the packing holder 620 may be manufactured using a double injection method.

Referring to FIGS. 1 to 3, the analyte monitoring device may further include a lower housing 700.

The lower housing 700 may be coupled to the upper housing 100 and may include at least one second opening 710 which is aligned with the first opening 110 and through which part of the sensor passes. When the sensor unit 200 is mounted, the sensor unit 200 may pass through the second opening 710 formed in the lower housing 700 so that at least the sensor insertion portion 220 can be exposed to the outside. The lower housing 700 may be a surface that contacts the body and may be manufactured of a material that is deformed to fit the body, and the outer surface of the lower housing 700 is implemented as an adhesive surface with an adhesive applied so that the analyte monitoring device can be attached to the surface of the body.

Referring to FIGS. 3 and 5, the analyte monitoring device may further include a battery 800 and a battery connection member 810.

The battery 800 may be coupled to the PCB unit 300 and may supply power to the analyte monitoring device. A circular battery is shown in FIGS. 3 and 5, but the present disclosure is not limited thereto, and a battery may be implemented in various shapes.

The battery connection member 810 may be coupled to the battery 800 and the PCB unit 300 and may supply power of the battery 800 to the PCB unit 300. The battery connection member 810 is not limited to the shape shown in FIG. 3 but may be implemented in various shapes.

### [Explanation of reference numerals]

| | | | |
|---|---|---|---|
| 100: | upper housing | 110: | first opening |
| 200: | sensor unit | 210: | sensor body portion |
| 220: | sensor insertion portion | 300: | PCB unit |
| 310: | PCB pad | 400: | antenna unit |
| 500: | conduction unit | 510: | antenna contact portion |
| 520: | PCB contact portion | 530: | conduction connection portion |
| 600: | fixing unit | 610: | holder |
| 611: | holder sensor fixing portion | 612: | holder insertion portion |
| 621: | packing holder sensor fixing portion | 622: | packing holder protrusion |
| 620: | packing holder | 700: | lower housing |
| 710: | second opening | 800: | battery |
| 810: | battery connection member | | |

## Claims

1. An analyte monitoring device comprising:
an upper housing;
a sensor unit inserted into a body;
a printed circuit board (PCB) unit which is spaced apart from an upper housing and on which the sensor unit is mounted;
an antenna unit formed on the upper housing; and
a conduction unit disposed between the PCB unit and the antenna unit and allowing the PCB unit and the antenna unit to be electrically connected to each other.

2. The analyte monitoring device of claim 1, wherein a first opening is formed in the upper housing, and a fixing unit coupled to the sensor unit is fixed in the first opening, and the fixing unit comprises a holder covering part of the first opening and coupled to the sensor unit, and a packing holder covering the other part of the first opening and coupled to the holder.

3. The analyte monitoring device of claim 2, wherein the packing holder comprises a packing holder sensor fixing portion formed with a width corresponding to a width of the sensor unit, and the holder comprises a holder sensor fixing portion formed with a width corresponding to the width of the sensor unit.

4. The analyte monitoring device of claim 3, wherein the packing holder further comprises a packing holder protrusion formed at one end of the packing holder in a protrusion structure, and the holder further comprises a holder insertion portion in a structure corresponding to the packing holder protrusion.

5. The analyte monitoring device of claim 2, wherein the packing holder is manufactured of a rubber material.

6. The analyte monitoring device of claim 1, wherein predetermined patterns are formed on the antenna unit using a laser direct structuring (LDS) method.

7. The analyte monitoring device of claim 1, wherein the sensor unit comprises:
a sensor body portion coupled to the PCB unit; and
a sensor insertion portion vertically extending from the sensor body portion and inserted into the body.

8. The analyte monitoring device of claim 1, wherein the conduction unit comprises:
an antenna contact portion in contact with the antenna unit;
a PCB contact portion disposed parallel to the antenna contact portion and in contact with the PCB unit; and
a conduction connection portion connecting the antenna contact portion to the PCB contact portion.

9. The analyte monitoring device of claim 1, further comprising a lower housing coupled to the upper housing, aligned with the first opening and comprising at least one second opening through which part of the sensor unit passes.
